# FASCICULE DE BREVET EUROPEEN

(11) **EP 4 076 602 B1**
(45) Date de publication et mention de la délivrance du brevet: **13.09.2023**
(21) Numéro de dépôt: 20845584.0
(22) Date de dépôt: 15.12.2020
(51) Int. Cl.: A61M 11/00, A61M 15/08, B05B 11/02

(54) **DISPOSITIF DE DISTRIBUTION DE PRODUIT FLUIDE**
VORRICHTUNG ZUR AUSGABE EINES FLUIDPRODUKTS
DEVICE FOR DISPENSING A FLUID PRODUCT

(30) Priorité: 19.12.2019 FR 1915029
(43) Date de publication de la demande: 26.10.2022
(73) Titulaire: APTAR France SAS, 27110 Le Neubourg (FR)
(72) Inventeur: BAILLIE, Mickaël, 27400 ACQUIGNY (FR); HUPPÉ, Maxime, 76320 CAUDEBEC LES ELBEUF (FR); NORRANT, Matthieu, 27120 HOULBEC COCHEREL (FR)
(74) Mandataire: CAPRI
(86) Numéro de dépôt international: PCT/FR2020/052456
(87) Numéro de publication internationale: WO 2021/123611

(56) Documents cités:
- EP-A1- 0 546 607
- WO-A1-2013/154954
- WO-A1-2016/097603
- WO-A2-01/93926
- WO-A2-2008/091838
- CN-B- 102 626 533
- US-A1- 2009 128 330
- US-A1- 2016 193 408
- US-A1- 2017 257 436

## Description

La présente invention concerne un dispositif de distribution de produit fluide, et plus particulièrement un dispositif du type unidose ou bidose, adapté à distribuer une ou deux doses de produit fluide en un seul actionnement.

Les dispositifs du type unidoses ou bidoses sont bien connus. Ils comportent généralement un réservoir contenant une ou deux doses de produit fluide, et une tête de distribution pourvue d'un orifice de distribution, et qui est déplaçable axialement par rapport audit réservoir lors de l'actionnement. Les documents EP0546607 et WO2016097603 décrivent des dispositifs de ce type.

Pour certains médicaments sensibles, l'utilisation du dispositif et donc la distribution d'une dose du médicament peut indiquer une situation d'urgence. Par exemple, dans le cas des médicaments à base de Naloxone, qui sont notamment utilisés pour se remettre d'un surdosage (pour les toxicomanes mais aussi pour les utilisateurs d'opioïdes « normaux »), la durée d'activité du médicament est très limitée, d'où la nécessité de consulter rapidement un médecin ou similaire pour obtenir une autre dose une fois nécessaire. Un autre exemple concerne l'épinéphrine, où il est aussi important que le patient se rende rapidement à l'hôpital pour un suivi, car l'efficacité de l'épinéphrine n'est que de quelques heures. Il peut en être de même avec d'autres médicaments, tels que par exemple le Fentanyl.

Les documents WO2008091838, US2016193408 et WO0193926 décrivent des dispositifs qui détectent l'actionnement d'un dispositif de distribution de médicament, et qui sont aptes à émettre une alerte, par exemple vers un numéro d'appel d'urgence. Ces dispositifs permettent d'alerter lorsque le dispositif est utilisé, mais si cette utilisation a été réussie, et que la dose a été correctement distribuée, l'alerte vers un numéro d'appel d'urgence n'est en réalité pas utile. Une telle alerte est par contre hautement souhaitable si l'utilisateur tente d'actionner le dispositif, mais n'y arrive, par exemple par manque de force, perte de connaissance, ou dysfonctionnement du dispositif.

Les documents CN102626533, US2017257436, WO2013154954 et US2009128330 décrivent d'autres dispositifs de l'état de la technique.

La présente invention a pour but de fournir un dispositif de distribution de produit fluide, notamment du type unidose ou bidose, qui ne reproduit pas les inconvénients susmentionnés.

La présente invention a aussi pour but de fournir un dispositif de distribution de produit fluide, notamment du type unidose ou bidose, qui appelle automatiquement un numéro d'urgence lors d'une tentative d'actionnement du dispositif.

La présente invention a également pour but de fournir un dispositif de distribution de produit fluide, notamment du type unidose ou bidose, qui est simple et peu coûteux à fabriquer et à assembler.

La présente a donc pour objet un dispositif de distribution de produit fluide comportant un corps et une tête de distribution pourvue d'un orifice de distribution et déplaçable axialement par rapport audit corps lors de l'actionnement, ledit corps recevant un réservoir contenant une ou deux doses de produit fluide, ledit dispositif comportant un module électronique qui comprend un module de communication sans fil, tel qu'un module GSM et/ou un module Wifi et/ou un module Bluetooth^{®}, un module de localisation géographique, tel qu'un module GPS et/ou un réseau d'antennes, et une source d'alimentation, telle qu'une batterie, ledit dispositif comportant un système de capteurs pour détecter et signaler automatiquement une prise en main représentative d'une tentative d'utilisation dudit dispositif, ledit système de capteurs comportant au moins un premier capteur disposé sur ladite tête de distribution et au moins un second capteur disposé sur ledit corps, ledit système de capteur détectant une prise en main représentative d'une tentative d'utilisation dudit dispositif lorsque lesdits premier et second capteurs sont activés simultanément, ledit module de communication sans fil étant adapté à effectuer un appel d'urgence automatique vers un numéro d'urgence lorsque ledit système de capteur détecte une prise en main représentative d'une tentative d'utilisation dudit dispositif.

Avantageusement, ledit au moins un premier capteur est un capteur de micro courant, un capteur capacitif, un capteur infrarouge, un capteur de luminosité, un capteur audio, un capteur de température ou un capteur d'humidité.

Avantageusement, ledit au moins un second capteur est un capteur de micro courant, un capteur capacitif, un capteur infrarouge, un capteur de luminosité, un capteur audio, un capteur de température ou un capteur d'humidité.

Avantageusement, avant actionnement, ledit module électronique est éteint ou en mode veille, avec une consommation d'énergie nulle ou minimale.

Avantageusement, ledit module électronique est allumé ou réveillé par ledit système de capteur pour passer de son mode veille ou éteint à un mode actif.

Avantageusement, ladite tête de distribution comporte une bride radiale sur laquelle l'utilisateur place un ou plusieurs doigts lors de l'actionnement, ledit au moins un premier capteur étant disposé sur ladite bride radiale.

Avantageusement, ledit corps comporte une paroi axiale distale sur laquelle l'utilisateur place un ou plusieurs doigts, typiquement le pouce, lors de l'actionnement, ledit au moins un second capteur étant disposé sur ladite paroi axiale distale.

Avantageusement, ledit module électronique comporte des moyens de temporisation pour décaler l'appel d'urgence automatique de quelques secondes de la détection d'une tentative d'utilisation par le système de capteur.

Avantageusement, ledit réservoir comporte un tube creux ayant une ouverture axiale proximale fermée par un bouchon adapté à coulisser de manière étanche dans ledit tube lors de l'actionnement.

Avantageusement, ladite tête de distribution comportant une canule creuse reliée d'un côté audit orifice de distribution et pourvue de l'autre côté d'une pointe de perçage pourvue d'une ouverture pour percer ledit bouchon.

Selon une première variante avantageuse, ledit produit fluide est un liquide.

Selon une seconde variante avantageuse, ledit produit fluide est une poudre.

Avantageusement, ledit réservoir contient une seule dose de produit fluide, distribuée en un seul actionnement.

En variante, ledit réservoir contient deux doses de produit fluide, distribuées en deux actionnements successifs.

Ces caractéristiques et avantages et d'autres apparaîtront plus clairement au cours de la description détaillée suivante, faite en référence aux dessins joints, donnés à titre d'exemples non limitatifs, et sur lesquels :
la figure 1 est une vue schématique en section transversale d'un dispositif du type unidose selon un premier mode de réalisation avantageux, en position de repos, et
la figure 2 est une vue schématique, similaire à celle de la figure 1, d'un dispositif du type unidose selon un second mode de réalisation avantageux.

Plus particulièrement, la présente invention concerne d'une part un dispositif du type unidose, tel que par exemple celui divulgué dans le document EP0546607, et d'autre part un dispositif du type bidose, tel que par exemple celui divulgué dans le document WO2016097603.

Il est toutefois entendu que la présente invention ne se limite pas à ces types de dispositif, mais est au contraire applicable à tous les types de dispositifs de distribution de produit fluide, du type unidose ou bidose.

Dans la description, les termes "axial" et "radial" se réfèrent à l'axe longitudinale du dispositif. Les termes "proximal" et "distal" se réfère à l'orifice de distribution 3 formé dans la tête de distribution 2.

L'invention s'applique d'une part à des dispositifs du type unidose tel que ceux représentés sur les figures, dans lesquels la totalité de la dose de produit fluide contenue dans le dispositif est distribuée en un seul actionnement du dispositif, et d'autre part à des dispositifs du type bidose, dans lesquels le produit fluide contenu dans le dispositif est distribué en deux actionnements successifs du dispositif.

Le dispositif du type unidose représenté sur les figures comporte un corps 1 qui reçoit un réservoir 10 contenant une dose de produit fluide, et une tête de distribution 2, pourvue d'un orifice de distribution 3, qui est déplaçable axialement par rapport audit corps 1 lors de l'actionnement.

Avantageusement, ledit réservoir 10 est formé par un tube creux borgne 11, par exemple en verre, ayant une ouverture axiale proximale 12 fermée par un bouchon 20, par exemple en élastomère, adapté à coulisser de manière étanche dans ledit tube 11 lors de l'actionnement.

La tête de distribution 2 comporte généralement une canule ou aiguille 4, de forme généralement cylindrique, reliée d'un côté audit orifice de distribution 3, et pourvue de l'autre côté d'une pointe de perçage 5 adaptée à percer ledit bouchon 20 lors de l'actionnement, le bouchon 20 se déplaçant alors dans ledit réservoir 10 pour expulser la ou les doses de produit fluide à travers ladite canule 4 vers ledit orifice de distribution 3. La canule 4 peut être insérée dans un support de canule 9, qui lui-même peut être fixé dans ladite tête de distribution 2. Avantageusement, un profil de pulvérisation 35 peut être formé directement en amont de l'orifice de distribution 3, par exemple entre le fond de ladite tête de distribution 2 et l'extrémité axiale proximale dudit support de canule 9.

La tête de distribution 2 comporte une bride radiale 6 sur laquelle l'utilisateur place un ou plusieurs doigts lors de l'actionnement.

Typiquement, l'actionnement du dispositif est réalisé en plaçant un ou plusieurs doigts sur ladite bride radiale 6, et un doigt, typiquement le pouce, sur une paroi axiale distale 7 du corps 1, et en déplaçant axialement le corps 1 par rapport à la tête de distribution 2.

Le dispositif comporte par ailleurs un module électronique 100 connecté à un système de capteur 201, 202.

Le module électronique 100 comporte avantageusement un module de communication sans fil 101, tel qu'un module GSM, un module de localisation géographique 102, tel qu'un module GPS, et une source d'alimentation 103, telle qu'une batterie.

Au repos, le module électronique 100 est avantageusement en mode veille, avec une consommation d'énergie minimale. En variante, il pourrait être complètement éteint au repos, avec donc une consommation énergétique nulle. Le réveil dudit module électronique est de préférence réalisé par le système de capteur.

Le système de capteur comporte au moins deux capteurs 201, 202 destinés à détecter une prise en main de l'utilisateur représentative d'une volonté manifeste d'utilisation du dispositif. Ainsi, la présente invention ne détecte pas l'actionnement du dispositif, ni la simple ouverture de l'emballage, mais seulement une prise en main telle que réalisée lorsqu'on souhaite actionner le dispositif.

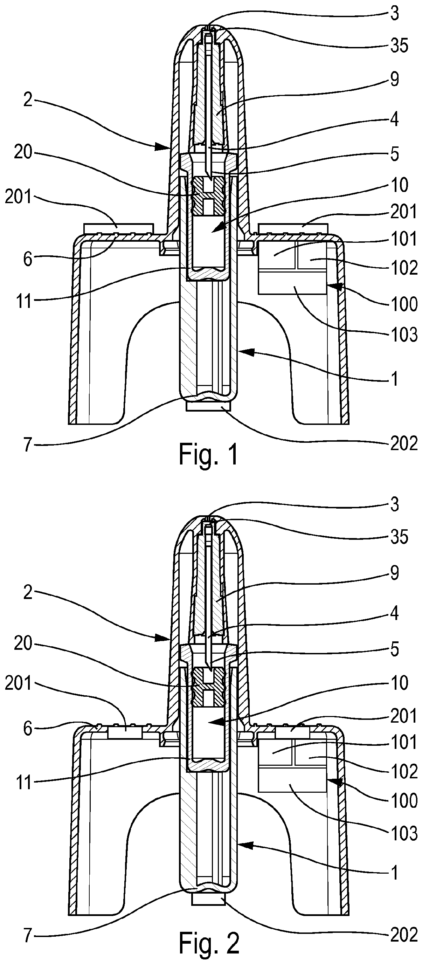

Selon l'invention, le système de capteur comporte au moins un premier capteur 201, disposé sur la bride radiale 6 de la tête de distribution 2. Dans l'exemple représenté, il y a deux premiers capteurs 201, diamétralement opposés sur ladite bride radiale 6. Cet au moins un premier capteur 201 détecte le ou les doigts que l'utilisateur pose sur ladite bride radiale 6 lorsqu'il souhaite actionner le dispositif. Ledit au moins un premier capteur 201, selon sa structure et son fonctionnement, peut être fixé sur la bride radiale 6, comme illustré sur la figure 1, ou inséré dans ladite bride radiale 6, comme illustré sur la figure 2.

Le système de capteur comporte également au moins un second capteur 202, disposé dans la paroi axiale distale 7 du corps 1. Dans l'exemple représenté, il y a un seul second capteur 202. Cet au moins un second capteur 202 détecte le ou les doigts, typiquement le pouce, que l'utilisateur pose sur ladite paroi axiale distale 7 lorsqu'il souhaite actionner le dispositif. Ledit au moins un second capteur 201, selon sa structure et son fonctionnement, peut également être fixé sur la paroi axiale distale 7, comme illustré sur la figure 1. Il pourrait aussi être inséré dans ladite paroi axiale distale 7.

L'association d'au moins un premier capteur 201 sur la bride radiale 6 et d'au moins un second capteur 202 sur la paroi axiale distale 7 permet de détecter une prise en main du dispositif qui est représentative d'une tentative d'utilisation. Ainsi, si seul l'un parmi le premier et le second capteur 201, 202 détecte la présence d'un doigt mais pas l'autre capteur, alors l'alerte n'est pas générée. Par contre, l'activation d'un seul des premiers et seconds capteurs permet avantageusement de "réveiller" le module électronique 100, pour le faire passer de son mode veille en mode actif.

Avantageusement, la détection d'une tentative d'utilisation n'est validée que si le système de capteur détecte l'activation simultanée d'un premier capteur 201 et d'un second capteur 202 pendant un temps minimum prédéterminé, par exemple au moins une seconde. Ceci permet d'éliminer les contacts furtifs non représentatifs d'une volonté d'utilisation du dispositif.

Plusieurs modes de réalisation du système de capteurs sont possibles.

Selon une première variante, les premiers et seconds capteurs 201, 202 sont des capteurs de micro courant. Dans cette première variante, chaque capteur comporte un pôle plus et un pôle moins, et un micro courant est généré lorsqu'un doigt contacte le premier capteur 201 et un autre doigt contacte le second capteur 202.

En variante, les premiers et seconds capteurs 201, 202 sont des capteurs capacitifs. Dans cette seconde variante aussi, on détecte une prise en main dite "en pince", avec au moins un doigt sur le premier capteur 201 de la bride radiale 6 et au moins un doigt sur le second capteur 202 sur la paroi axiale distale 7.

Selon encore d'autres variantes, les premiers et seconds capteurs 201, 202 peuvent être des capteurs infrarouges, des capteurs de luminosité, des capteurs audio, des capteurs de températures ou des capteurs d'humidité. Eventuellement, une combinaison de capteurs est possible, le premier capteur 201 n'étant pas nécessairement identique au second capteur 202.

Lorsque le système de capteur détecte une tentative d'utilisation, le module GPS 102 va alors établir la position géographique du dispositif et le module GSM 101 va effectuer un appel d'urgence automatique vers un numéro d'urgence, tel que par exemple le 110 en Europe, le 15 ou le 18 en France ou le 911 aux USA.

Dans une variante avantageuse, l'appel d'urgence automatique est décalé de quelques secondes de la détection d'une tentative d'utilisation par le système de capteur, par exemple par des moyens de temporisation prévus dans le module électronique 100. Cette mise en oeuvre permet de désactiver l'appel d'urgence si dans cet intervalle le dispositif est effectivement actionné par l'utilisateur. Dans cette hypothèse, le dispositif comporterait un troisième capteur capable de détecter l'actionnement, par exemple le déplacement axial du corps 1 par rapport à la tête de distribution 2.

L'appel d'urgence automatique peut comporter la transmission d'un message préenregistré accompagné de la position géographique déterminée par le module GPS 102. L'appel peut aussi comporter un code pour identifier automatiquement le médicament disposé dans le dispositif de distribution de produit fluide. Avantageusement, il pourrait être prévu une codification pour chaque médicament sensible auquel la présente invention s'adresse plus particulièrement. L'appel peut aussi comporter une identification de l'utilisateur ainsi qu'un horodatage.

En variante, l'appel peut mettre l'utilisateur en contact avec le service d'urgence, de sorte que l'utilisateur peut directement communiquer avec ledit service d'urgence.

Selon une première variante, le module GSM 101 peut effectuer des appels d'urgence jusqu'à ce que la batterie 103 soit vide. Selon une seconde variante, le nombre d'appel peut être prédéfini. Selon une troisième variante, le dispositif ne réalise qu'un seul appel.

Après le ou les appels, le module électronique 100 peut retourner en mode veille ou s'éteindre.

Le module de communication sans fil 101 pourrait en variante comporter un module Wifi et/ou un module Bluetooth^{®}, et plus généralement tout type de moyens de communication sans fil utilisant diverses largeurs de bande et/ou fréquences.

Le module de localisation géographique 102 pourrait en variante comporter un module Galileo, et plus généralement tout type de moyens de localisation géographique. Ainsi, le module de localisation géographique pourrait ne pas utiliser de liaison satellite, mais un réseau d'antennes, par exemple via un réseau GSM ou Wifi ou similaire. Une combinaison des deux moyens de localisation, par satellite et par antenne, est aussi envisageable.

La source d'alimentation 103 pourrait en variante être réalisée sous la forme d'un accumulateur rechargeable. Par ailleurs, un condensateur, ou tout moyen similaire, peut être associé à la source d'alimentation 103 pour permettre de délivrer, en cas de besoin, un courant élevé aux différents modules décrits ci-dessus.

La présente invention a été décrite en référence à plusieurs modes de réalisation avantageux, mais il est entendu qu'un homme du métier peut y apporter toutes modifications, sans sortir du cadre de la présente invention tel que défini par les revendications annexées.

## Revendications

1. Dispositif de distribution de produit fluide comportant un corps (1) et une tête de distribution (2) pourvue d'un orifice de distribution (3) et déplaçable axialement par rapport audit corps (1) lors de l'actionnement, ledit corps (1) recevant un réservoir (10) contenant une ou deux doses de produit fluide, ledit dispositif comportant un module électronique (100) qui comprend un module de communication sans fil (101), tel qu'un module GSM et/ou un module Wifi et/ou un module Bluetooth^{®}, un module de localisation géographique (102), tel qu'un module GPS et/ou un réseau d'antennes, et une source d'alimentation (103), telle qu'une batterie, **caractérisé en ce que** ledit dispositif comporte un système de capteurs (201, 202) pour détecter et signaler automatiquement une prise en main représentative d'une tentative d'utilisation dudit dispositif, ledit système de capteurs comportant au moins un premier capteur (201) disposé sur ladite tête de distribution (2) et au moins un second capteur (202) disposé sur ledit corps (1), ledit système de capteur détectant une prise en main représentative d'une tentative d'utilisation dudit dispositif lorsque lesdits premier et second capteurs (201, 202) sont activés simultanément, ledit module de communication sans fil (101) étant adapté à effectuer un appel d'urgence automatique vers un numéro d'urgence lorsque ledit système de capteur (201, 202) détecte une prise en main représentative d'une tentative d'utilisation dudit dispositif.

2. Dispositif selon la revendication 1, dans lequel ledit au moins un premier capteur (201) est un capteur de micro courant, un capteur capacitif, un capteur infrarouge, un capteur de luminosité, un capteur audio, un capteur de température ou un capteur d'humidité.

3. Dispositif selon la revendication 1 ou 2, dans lequel ledit au moins un second capteur (202) est un capteur de micro courant, un capteur capacitif, un capteur infrarouge, un capteur de luminosité, un capteur audio, un capteur de température ou un capteur d'humidité.

4. Dispositif selon l'une quelconque des revendications précédentes, dans lequel, avant actionnement, ledit module électronique (100) est éteint ou en mode veille, avec une consommation d'énergie nulle ou minimale.

5. Dispositif selon la revendication 4, dans lequel ledit module électronique est allumé ou réveillé par ledit système de capteur (201, 202) pour passer de son mode veille ou éteint à un mode actif.

6. Dispositif selon l'une quelconque des revendications précédentes, dans lequel ladite tête de distribution (2) comporte une bride radiale (6) sur laquelle l'utilisateur place un ou plusieurs doigts lors de l'actionnement, ledit au moins un premier capteur (201) étant disposé sur ladite bride radiale (6).

7. Dispositif selon l'une quelconque des revendications précédentes, dans lequel ledit corps (1) comporte une paroi axiale distale (7) sur laquelle l'utilisateur place un ou plusieurs doigts, typiquement le pouce, lors de l'actionnement, ledit au moins un second capteur (202) étant disposé sur ladite paroi axiale distale (7).

8. Dispositif selon l'une quelconque des revendications précédentes, dans lequel ledit module électronique (100) comporte des moyens de temporisation pour décaler l'appel d'urgence automatique de quelques secondes de la détection d'une tentative d'utilisation par le système de capteur.

9. Dispositif selon l'une quelconque des revendications précédentes, dans lequel ledit réservoir (10) comporte un tube creux (11) ayant une ouverture axiale proximale (12) fermée par un bouchon (20) adapté à coulisser de manière étanche dans ledit tube (11) lors de l'actionnement.

10. Dispositif selon l'une quelconque des revendications précédentes, dans lequel ladite tête de distribution (2) comportant une canule creuse (4) reliée d'un côté audit orifice de distribution (3) et pourvue de l'autre côté d'une pointe de perçage (5) pourvue d'une ouverture (6) pour percer ledit bouchon (20).

11. Dispositif selon l'une quelconque des revendications précédentes, dans lequel ledit produit fluide est un liquide.

12. Dispositif selon l'une quelconque des revendications 1 à 10, dans lequel ledit produit fluide est une poudre.

13. Dispositif selon l'une quelconque des revendications précédentes, dans lequel ledit réservoir (10) contient une seule dose de produit fluide, distribuée en un seul actionnement.

14. Dispositif selon l'une quelconque des revendications 1 à 12, dans lequel ledit réservoir (10) contient deux doses de produit fluide, distribuées en deux actionnements successifs.

## Patentansprüche

1. Vorrichtung zur Abgabe eines Fluidprodukts, die einen Körper (1) und einen Abgabekopf (2) aufweist, der mit einer Abgabeöffnung (3) versehen ist und bei der Betätigung im Verhältnis zum Körper (1) axial bewegbar ist, wobei der Körper (1) einen Vorratsbehälter (10) aufnimmt, der eine oder zwei Fluidproduktdosen enthält, wobei die Vorrichtung ein elektronisches Modul (100) aufweist, das ein Drahtloskommunikationsmodul (101) wie etwa ein GSM-Modul und/oder ein Wifi-Modul und/oder ein Bluetooth^{®}-Modul, ein Modul zur geografischen Lokalisierung (102) wie etwa ein GPS-Modul und/oder ein Antennennetzwerk und eine Stromversorgungsquelle (103) wie etwa eine Batterie umfasst, **dadurch gekennzeichnet, dass** die Vorrichtung ein System von Sensoren (201, 202) zur Detektion und automatischen Signalisierung einer Indiehandnahme aufweist, die für einen Versuch der Benutzung der Vorrichtung repräsentativ ist, wobei das System von Sensoren wenigstens einen ersten Sensor (201), der auf dem Abgabekopf (2) angeordnet ist, und wenigstens einen zweiten Sensor (202) aufweist, der auf dem Körper (1) angeordnet ist, wobei das Sensorsystem eine Indiehandnahme detektiert, die für einen Versuch der Benutzung der Vorrichtung repräsentativ ist, wenn der erste und zweite Sensor (201, 202) gleichzeitig aktiviert werden, wobei das Drahtloskommunikationsmodul (101) geeignet ist, einen automatischen Notruf einer Notrufnummer zu tätigen, wenn das Sensorsystem (201, 202) eine Indiehandnahme detektiert, die für einen Versuch der Benutzung der Vorrichtung repräsentativ ist.

2. Vorrichtung nach Anspruch 1, wobei der wenigstens eine erste Sensor (201) ein Mikrostromsensor, ein kapazitiver Sensor, ein Infrarotsensor, ein Helligkeitssensor, ein Audiosensor, ein Temperatursensor oder ein Feuchtesensor ist.

3. Vorrichtung nach Anspruch 1 oder 2, wobei der wenigstens eine zweite Sensor (202) ein Mikrostromsensor, ein kapazitiver Sensor, ein Infrarotsensor, ein Helligkeitssensor, ein Audiosensor, ein Temperatursensor oder ein Feuchtesensor ist.

4. Vorrichtung nach einem der vorhergehenden Ansprüche, wobei vor Betätigung das elektronische Modul (100) ausgeschaltet oder im Bereitschaftsmodus ist, mit einem Energieverbrauch von null oder einem minimalen Energieverbrauch.

5. Vorrichtung nach Anspruch 4, wobei das elektronische Modul vom Sensorsystem (201, 202) eingeschaltet oder aufgeweckt wird, um von seinem Bereitschafts- oder ausgeschalteten Modus in einen aktiven Modus zu wechseln.

6. Vorrichtung nach einem der vorhergehenden Ansprüche, wobei der Abgabekopf (2) einen Radialflansch (6) aufweist, auf den der Benutzer bei der Betätigung einen oder mehrere Finger legt, wobei der wenigstens eine erste Sensor (201) auf dem Radialflansch (6) angeordnet ist.

7. Vorrichtung nach einem der vorhergehenden Ansprüche, wobei der Körper (1) eine distale Axialwand (7) aufweist, auf die der Benutzer bei der Betätigung einen oder mehrere Finger legt, typischerweise den Daumen, wobei der wenigstens eine zweite Sensor (202) auf der distalen Axialwand (7) angeordnet ist.

8. Vorrichtung nach einem der vorhergehenden Ansprüche, wobei das elektronische Modul (100) Verzögerungsmittel aufweist, um den automatischen Notruf ab der Detektion eines Versuchs der Benutzung durch das Sensorsystem um einige Sekunden zu verschieben.

9. Vorrichtung nach einem der vorhergehenden Ansprüche, wobei der Vorratsbehälter (10) ein Hohlrohr (11) mit einer proximalen Axialöffnung (12) aufweist, die von einem Stopfen (20) verschlossen ist, der geeignet ist, bei der Betätigung dichtend im Rohr (11) zu gleiten.

10. Vorrichtung nach einem der vorhergehenden Ansprüche, wobei der Abgabekopf (2) eine hohle Kanüle (4) aufweist, die auf einer Seite mit der Abgabeöffnung (3) verbunden ist und auf der anderen Seite mit einer Durchstechspitze (5) versehen ist, die mit einer Öffnung (6) versehen ist, um den Stopfen (20) zu durchstechen.

11. Vorrichtung nach einem der vorhergehenden Ansprüche, wobei das Fluidprodukt eine Flüssigkeit ist.

12. Vorrichtung nach einem der Ansprüche 1 bis 10, wobei das Fluidprodukt ein Pulver ist.

13. Vorrichtung nach einem der vorhergehenden Ansprüche, wobei der Vorratsbehälter (10) eine einzelne Fluidproduktdosis enthält, die mit einer einzelnen Betätigung abgegeben wird.

14. Vorrichtung nach einem der Ansprüche 1 bis 12, wobei der Vorratsbehälter (10) zwei Fluidproduktdosen enthält, die mit zwei aufeinanderfolgenden Betätigungen abgegeben werden.

## Claims

1. Device for dispensing a fluid product comprising a body (1) and a dispensing head (2) provided with a dispensing orifice (3) and axially movable with respect to said body (1) during actuation, said body (1) receiving a vessel (10) containing one or two doses of fluid product, said device comprising an electronic module (100) which comprises a wireless communication module (101), such as a GSM module and/or a Wi-Fi module and/or a Bluetooth^{®} module, a geographical location module (102), such as a GPS module and/or an antenna array, and a power source (103), such as a battery, **characterised in that** said device comprises a sensor system (201, 202) for automatically detecting and signalling handling representative of an attempt to use said device, said sensor system comprising at least one first sensor (201) arranged on said dispensing head (2) and at least one second sensor (202) arranged on said body (1), said sensor system detecting handling representative of an attempt to use said device when said first and second sensors (201, 202) are activated simultaneously, said wireless communication module (101) being designed to make an automatic emergency call to an emergency number when said sensor system (201, 202) detects handling representative of an attempt to use said device.

2. Device according to claim 1, wherein said at least one first sensor (201) is a microcurrent sensor, a capacitive sensor, an infrared sensor, a luminosity sensor, an audio sensor, a temperature sensor or a humidity sensor.

3. Device according to claim 1 or 2, wherein said at least one second sensor (202) is a microcurrent sensor, a capacitive sensor, an infrared sensor, a luminosity sensor, an audio sensor, a temperature sensor or a humidity sensor.

4. Device according to any one of the preceding claims, wherein, before actuation, said electronic module (100) is switched off or in standby mode, with a zero or minimum energy consumption.

5. Device according to claim 4, wherein said electronic module is switched on or awakened by said sensor system (201, 202) to go from its standby or switched off mode to an active mode.

6. Device according to any one of the preceding claims, wherein said dispensing head (2) includes a radial flange (6) on which the user places one or more fingers during actuation, said at least one first sensor (201) being disposed on said radial flange (6).

7. Device according to any one of the preceding claims, wherein said body (1) includes a distal axial wall (7) on which the user places one or more fingers, typically the thumb, during actuation, said at least one second sensor (202) being disposed on said distal axial wall (7).

8. Device according to any one of the preceding claims, wherein said electronic module (100) comprises timeout means to postpone the automatic emergency call by a few seconds from detecting an attempt to use by the sensor system.

9. Device according to any one of the preceding claims, wherein said vessel (10) comprises a hollow tube (11) having a proximal axial opening (12) that is closed by a stopper (20) that is adapted to slide in leaktight manner in said tube (11) during actuation.

10. Device according to any one of the preceding claims, wherein said dispensing head (2) comprises a hollow cannula (4) connected on one side to said dispensing orifice (3) and provided on the other side with a perforator tip (5) provided with an opening (6) for piercing said stopper (20).

11. Device according to any one of the preceding claims, wherein said fluid product is a liquid.

12. Device according to any one of claims 1 to 10, wherein said fluid product is a powder.

13. Device according to any one of the preceding claims, wherein said vessel (10) contains one single dose of fluid product, dispensed in one single actuation.

14. Device according to any one of claims 1 to 12, wherein said vessel (10) contains two doses of fluid product, dispensed in two successive actuations.
